# EUROPÄISCHE PATENTSCHRIFT

(11) **EP 2 672 847 B1**
(45) Veröffentlichungstag und Bekanntmachung des Hinweises auf die Patenterteilung: **22.04.2015**
(21) Anmeldenummer: 12712527.6
(22) Anmeldetag: 02.02.2012
(51) Int. Cl.: A24F 47/00, A61M 15/06, B05B 1/24, H05K 1/02

(54) **INHALATORKOMPONENTE**
INHALER COMPONENT
ÉLÉMENT INHALATEUR

(30) Priorität: 11.02.2011 AT 1872011; 27.07.2011 AT 10952011
(43) Veröffentlichungstag der Anmeldung: 18.12.2013
(73) Patentinhaber: Batmark Limited, London WC2R 2PG (GB)
(72) Erfinder: Buchberger, Helmut, 4482 Ennsdorf (AT)
(74) Vertreter: Schrell, Andreas
(86) Internationale Anmeldenummer: PCT/AT2012/000017
(87) Internationale Veröffentlichungsnummer: WO 2012/106739

(56) Entgegenhaltungen:
- EP-A1- 0 893 071
- EP-A1- 2 018 886
- WO-A1-2010/045671

## Beschreibung

Die Erfindung betrifft eine Inhalatorkomponente für die Bildung eines Dampf-Luft-Gemisches oder/und Kondensationsaerosols durch Verdampfung eines flüssigen Materials und gegebenenfalls Kondensation des gebildeten Dampfes, umfassend:
ein Heizelement zur Verdampfung einer Portion des flüssigen Materials;
einen Docht zur selbsttätigen Versorgung des Heizelements mit dem flüssigen Material, welcher Docht zumindest zwei voneinander entfernt angeordnete Endabschnitte aufweist;
einen ersten Kapillarspalt zur selbsttätigen Versorgung des Dochts mit dem flüssigen Material, wobei ein erster Endabschnitt des Dochts in den ersten Kapillarspalt ragt.

### Begriffsdefinition:

In der gegenständlichen Patentanmeldung bezieht sich der Begriff "Inhalator" auf medizinische wie nicht-medizinische Inhalatoren. Der Begriff bezieht sich ferner auf Inhalatoren zur Verabreichung von Arzneimitteln und solchen Stoffen, welche nicht als Arzneimittel deklariert sind. Der Begriff bezieht sich außerdem auf Rauchartikel und Zigarettenersatz-Artikel, wie sie beispielsweise in der Europäische Patentklasse A24F47/00B enthalten sind, soweit diese dazu bestimmt sind, dem Benutzer ein Dampf-Luft-Gemisch oder/und Kondensationsaerosol darzureichen. Der Begriff "Inhalator" soll auch keine Einschränkungen dahingehend machen, wie das gebildete Dampf-Luft-Gemisch oder/und Kondensationsaerosol dem Benutzer bzw. dessen Körper zugeführt wird. Das Dampf-Luft-Gemisch oder/und Kondensationsaerosol kann in die Lunge inhaliert werden, oder aber auch nur der Mundhöhle zugeführt werden - ohne Inhalation in die Lunge.

Als "Kapillarspalt" gelte ein jeder Spalt, welcher allein aufgrund der Kapillarwirkung seiner Begrenzungswände einen Flüssigkeitstransport bewirkt.

Dochte, ummantelte Dochte oder mit Dochtmaterial befüllte Kanäle sind keine Kapillarspalten.

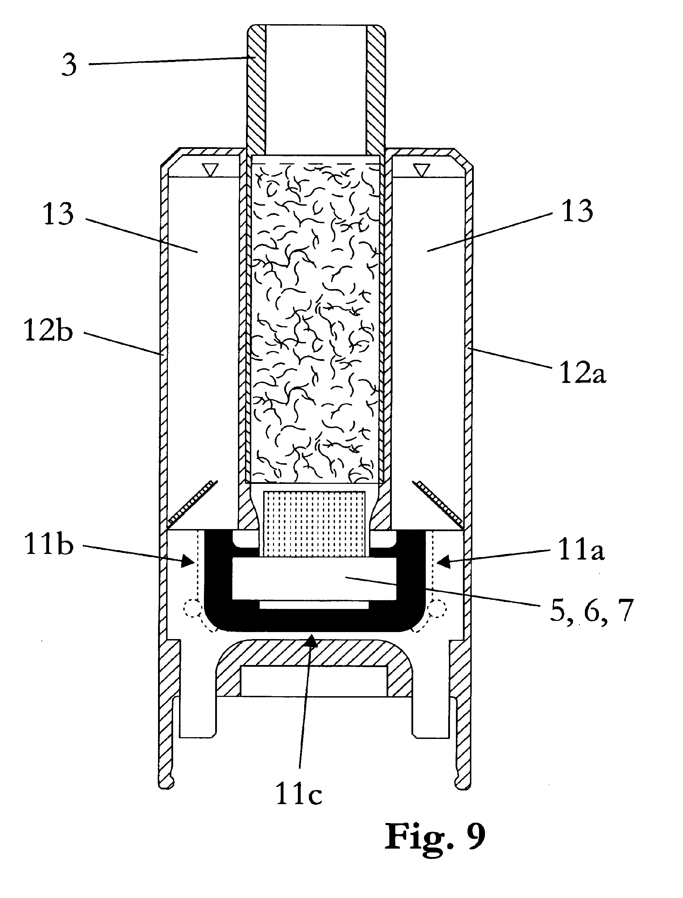

WO 2010/045671 (Helmut Buchberger) beschreibt eine Inhalatorkomponente für die intermittierende, inhalations- oder zugsynchrone Bildung eines Dampf-Luft-Gemisches oder/und Kondensationsaerosols, bestehend aus (Fig. 9-12 und Fig. 17-18) einem Gehäuse 3, eine im Gehäuse 3 angeordnete Kammer 21, eine Lufteinlaßöffnung 26 für die Zufuhr von Luft aus der Umgebung in die Kammer 21, ein elektrisches Heizelement zur Verdampfung einer Portion eines flüssigen Materials 16, wobei der gebildete Dampf sich in der Kammer 21 mit der durch die Lufteinlaßöffnung 26 zugeführten Luft mischt, und sich das Dampf-Luft-Gemisch oder/und Kondensationsaerosol bildet. Die Inhalatorkomponente umfaßt ferner einen Docht mit einer Kapillarstruktur, welcher Docht mit dem Heizelement einen flächigen Verbund 22 bildet und das Heizelement nach einer Verdampfung von neuem selbsttätig mit dem flüssigen Material 16 versorgt. Zumindest ein beheizter Abschnitt des Verbundes 22 ist berührungsfrei in der Kammer 21 angeordnet, und die Kapillarstruktur des Dochts liegt im besagten Abschnitt wenigstens auf einer Seite 24 des flächigen Verbundes weitgehend frei. Der flächige Verbund 22 ragt mit einem Ende in einen Kapillarspalt 41, welcher seinerseits mit einem das flüssige Material 16 enthaltenden Flüssigkeitsbehälter 4 kapillar gekoppelt bzw. koppelbar ist. Der Kapillarspalt 41 zieht das flüssige Material 16 aus dem Flüssigkeitsbehälter 4 und transportiert es zum Docht.

Nach einer Verdampfung bzw. Inhalation muß der Benutzer der Inhalatorkomponente eine Wartezeit einhalten, während welcher das flüssige Material 16 den Doch von neuem vollständig infiltrieren kann. Verdampfungen vor Ablauf der Wartezeit können verschiedene ungünstige Folgen nach sich ziehen, beispielsweise eine Verringerung der verabreichten Aerosolmenge oder/und eine lokale Überhitzung des Dochts gegebenenfalls verbunden mit einer Zersetzung des flüssigen Materials und einer Verschlechterung der organoleptischen Eigenschaften des gebildeten Dampf-Luft-Gemisches bzw. Aerosols. In Prototypen auf Basis von hochverdünnten ethanolischen oder/und wässrigen Nikotinlösungen konnte eine vollständige Infiltration des Dochts innerhalb von 10s erzielt werden. Soll die Inhalatorkomponente als Zigarettenersatz Verwendung finden, so mag eine Wartezeit von 10s für viele Raucher akzeptabel sein; für manche Raucher mag sie allerdings zu lange sein. Ferner hat sich bei denselbigen Prototypen gezeigt, daß auch bei Einhaltung der besagten Wartezeit Störungen der Infiltration auftreten können. Diese Störungen treten zwar selten auf, können aber dieselbigen ungünstigen Folgen nach sich ziehen wie zuvor beschrieben. Die Störungen sind durch eine mangelhafte Benetzung der Kapillarstruktur des Dochts durch das flüssige Material gekennzeichnet und treten bevorzugt lokal, in in Bezug zum Kapillarspalt peripheren Bereichen des Dochts auf.

Der Erfindung liegt die Aufgabe zugrunde, die zuvor aufgezeigten Nachteile der aus dem Stand der Technik bekannten Anordnung zu beheben. Der Erfindung liegt insbesondere die Aufgabe zugrunde, eine Inhalatorkomponente der eingangs geschilderten Art so auszugestalten, daß der Docht vom flüssigen Material möglichst rasch infiltriert wird, und keine unangenehm langen Wartezeiten auftreten. Lokale Störungen der Infiltration sollen ebenfalls vermieden werden. Dies alles soll möglichst ohne zusätzlichen Bauaufwand erreicht werden. Die Fabrikationskosten der Inhalatorkomponente sollen sich ebenfalls nicht erhöhen.

Die Aufgabe wird durch die kennzeichnenden Merkmale des Patentanspruches 1 gelöst. Demnach weist die Inhalatorkomponente einen zweiten Kapillarspalt auf, welcher den zweiten Endabschnitt des Dochts in sich aufnimmt. Der Docht wird also von zwei Seiten her mit dem flüssigen Material versorgt. Dadurch kann die Wartezeit bis zur vollständigen Infiltration des Dochts im Vergleich zu einer konventionellen einseitigen Versorgung mindestens halbiert werden. Berücksichtigt man, daß die Infiltration des Dochts mit dem flüssigen Material degressivproportional, das heißt, zu Beginn vergleichsweise schnell und dann verzögert verläuft, dann wird klar, daß sich die Wartezeit bis zur vollständigen Infiltration des Dochts durch die erfindungsgemäße Anordung um deutlich mehr als 50% verkürzen läßt. Ähnlich günstige Effekte ergeben sich hinsichtlich der Versorgungssicherheit des Dochts: die besonders gefährdeten, in Bezug zum ersten Kapillarspalt peripheren Bereiche des Dochts können nun über kurze Distanz vom zweiten Kapillarspalt sicher mit dem flüssigen Material versorgt werden.

In einer bevorzugten Ausbildung der Erfindung ist vorgesehen, daß der erste und zweite Kapillarspalt über einen dritten Kapillarspalt miteinander verbunden sind. Der erste und zweite Kapillarspalt kommunizieren also über den dritten Kapillarspalt miteinander. Dadurch kann eine etwaige ungleichmäßige Versorgung des ersten und zweiten Kapillarspalts mit dem flüssigen Material ausgeglichen werden, und die Versorgungssicherheit des Dochts weiter verbessert werden.

Erfindungsgemäß ist ferner vorgesehen, daß einer der Kapillarspalten mit einem das flüssige Material enthaltenden Flüssigkeitsbehälter kapillar gekoppelt bzw. koppelbar ist. Dieser Kapillarspalt kann beispielsweise der erste Kapillarspalt sein. In diesem Fall würde der zweite Kapillarspalt ausschließlich über den dritten Kapillarspalt mit dem flüssigen Material versorgt. Alternativ könnte aber auch vorgesehen sein, daß der dritte Kapillarspalt mit dem Flüssigkeitsbehälter kapillar gekoppelt bzw. koppelbar ist. In diesem Fall würden der erste und der zweite Kapillarspalt über den dritten Kapillarspalt mit dem flüssigen Material versorgt.

Konstruktiv besonders einfache Verhältnisse ergeben sich, wenn sämtliche Kapillarspalten in einer gemeinsamen Ebene angeordnet sind. Eine weitere konstruktive Vereinfachung wird dadurch erzielt, daß sämtliche Kapillarspalten durch eine Platte, vorzugsweise Leiterplatte, und ein auf die Platte aufgesetztes Oberteil gebildet werden. In diesem Fall sind bloß zwei Bauteile erforderlich, um alle Kapillarspalten auszubilden.

In einer ersten Ausgestaltungsvariante weist das Oberteil erfindungsgemäß eine der Platte zugewandte Ausnehmung auf. Die Ausnehmung bildet im Zusammenwirken mit der Plattenoberfläche die Kapillarspalten aus, wobei die Tiefe der Ausnehmung die Spaltbreite der Kapillarspalten festlegt. Besonders günstig ist es, wenn die Ausnehmung zumindest abschnittsweise von einer oder mehreren Belüftungsnuten berandet wird. Die Belüftungsnuten haben den vorteilhaften Effekt, daß das in den Kapillarspalten gespeicherte flüssige Material effektiver als Puffervolumen genutzt werden kann.

In einer zweiten alternativen Ausgestaltungsvariante lagert das Oberteil auf den Endabschnitten des Dochts. Die Endabschnitte des Dochts wirken in diesem Fall als Abstandshalter, welche die Spaltbreite der Kapillarspalten festlegen. Auch in dieser alternativen Ausgestaltungsvariante gelte das Oberteil als "auf die Platte aufgesetzt", selbst wenn die beiden Bauteile einander nicht direkt berühren.

Die Platte ist vorzugsweise als Leiterplatte ausgebildet und dient als solche für die Zuleitung von elektrischer Energie zum in diesem Fall elektrischen Heizelement. Hierbei ist es besonders vorteilhaft, wenn die Leiterplatte als mehrlagige sogenannte Multilayer-Leiterplatte ausgeführt ist. Die den elektrischen Strom zuführenden Leiterbahnen können dadurch nämlich in Lagen konzentriert werden, welche die Kapillarspalten nicht tangieren. Ferner können mittels Multilayer-Leiterplatten auch komplexere Leiterbahn-Anordnungen realisiert werden, ein Umstand, welcher sich spätestens dann als vorteilhaft erweist, wenn mehrere elektrische Heizelemente vorgesehen sind, und die Heizelemente unabhängig voneinander angesteuert werden sollen. Schließlich erlauben Multilayer-Leiterplatten aufgrund der mehrlagigen Anordnung ihrer Leiterbahnen die Übertragung vergleichsweise hoher elektrischer Ströme.

In einer weiteren Ausgestaltung der Erfindung ist vorgesehen, daß der erste Kapillarspalt mit einem das flüssige Material enthaltenden ersten Flüssigkeitsbehälter gekoppelt bzw. koppelbar ist, und der zweite Kapillarspalt mit einem das flüssige Material enthaltenden zweiten Flüssigkeitsbehälter gekoppelt bzw. koppelbar ist. Durch das Vorsehen von zwei voneinander im Wesentlichen unabhängigen Flüssigkeitsbehältern kann die Versorgungssicherheit des Dochts mit dem flüssigen Material nochmals erhöht werden.

Zweckmäßige und vorteilhafte Ausführungsbeispiele der Erfindung sind in den Zeichnungen dargestellt und werden in der nachfolgenden Beschreibung näher erläutert.

Es zeigen:
Fig. 1 eine erfindungsgemäße Inhalatorkomponente in verschiedenen Ansichten;
Fig. 2 einen Längsschnitt durch die Inhalatorkomponente nach Fig. 1 in Höhe des flächigen Verbundes;
Fig. 3 eine Schnittansicht der Inhalatorkomponente längs der Linie A-A in Fig. 2;
Fig. 4 das Detail a aus Fig. 3 in einer vergrößerten Darstellung;
Fig. 5 eine Schnittansicht der Inhalatorkomponente längs der Linie B-B in Fig. 2;
Fig. 6 die Leiterplatte samt flächigem Verbund;
Fig. 7 die Leiterplatte samt flächigem Verbund zusammengefügt mit dem die Kapillarspalten ausbildenden Oberteil;
Fig. 8 das die Kapillarspalten ausbildende Oberteil in zwei Ansichten;
Fig. 9 eine erfindungsgemäße Inhalatorkomponente in einer alternativen Ausführungsform in einer Ansicht analog zu Fig. 2.

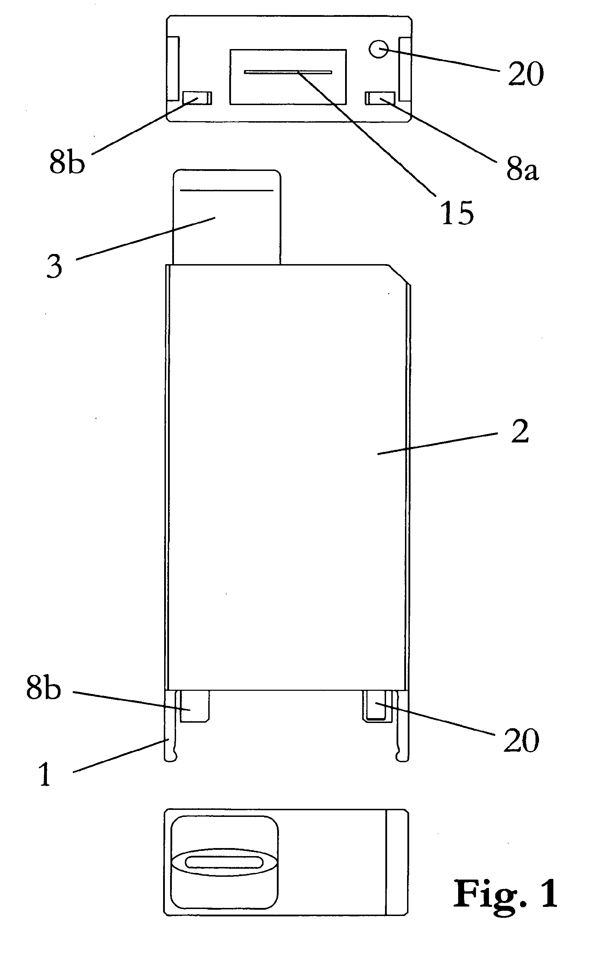

Fig. 1 zeigt ein erstes Ausführungsbeispiel einer erfindungsgemäßen Inhalatorkomponente. Die Inhalatorkomponente ist im konkreten Beispiel als auswechselbares Teil des Inhalators ausgebildet und über eine Schnappverbindung 1 mit einem nicht weiter dargestellten wiederverwendbaren Inhalatorteil koppelbar. Die Inhalatorkomponente bildet zusammen mit dem wiederverwendbaren Inhalatorteil den Inhalator. Die Inhalatorkomponente besteht aus einem Gehäuse 2 und umfaßt ferner ein Mundstück 3, über welches der Benutzer des Inhalators das Dampf-Luft-Gemisch oder/und Kondensationsaerosol bezieht.

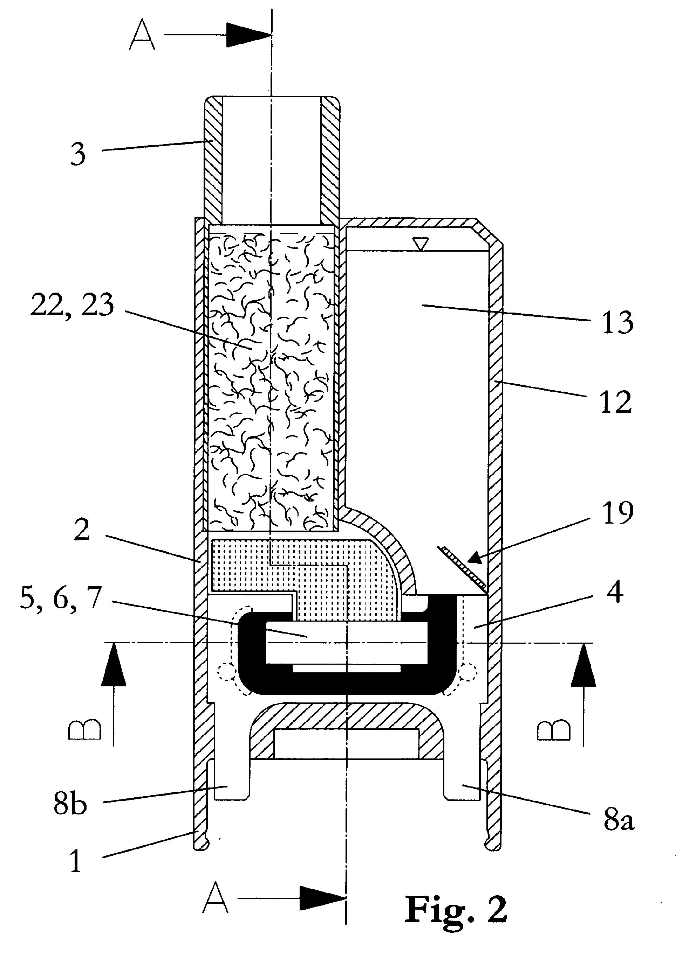

Die Fig. 2-5 geben weiteren Aufschluss über den inneren Aufbau der Inhalatorkomponente. Demnach befindet sich im Gehäuse 2 eine Trägerplatte 4, welche vorzugsweise als Leiterplatte ausgebildet ist. Die Leiterplatte 4 trägt einen flächigen Verbund 5. Der flächige Verbund 5 besteht aus einem Docht 7 und einem elektrischen Heizelement 6, welche miteinander flächig verbunden oder ineinander integriert sind. Der flächige Verbund 5 kann beispielsweise durch eine Metallfolie und darauf aufgesinterten Metallgewebelagen gebildet werden. Der flächige Verbund 5 kann alternativ auch aus einem offenporigen Metallschaum bestehen. Die offenporige Kapillarstruktur der auf die Metallfolie aufgesinterten Gewebelagen bzw. des Metallschaums bildet den Docht 7 aus, und der elektrische Widerstand des Metalls bildet das Heizelement 6 aus. Geeignete metallische Widerstandsmaterialien sind beispielsweise Edelstähle wie AISI 304 oder AISI 316 sowie Heizleiterlegierungen, insbesondere NiCr-Legierungen.

Der Docht 7 bzw. der diesen enthaltende flächige Verbund 5 weisen zwei voneinander entfernt angeordnete Endabschnitte 7a und 7b auf. Der flächige Verbund 5 lagert mit diesen Endabschnitten auf der Leiterplatte 4. Der flächige Verbund 5 ist ferner im Bereich der Endabschnitte 7a und 7b auf Leiterbahnen der Leiterplatte 4 elektrisch kontaktiert. Die elektrische Kontaktierung des flächigen Verbundes 5 bzw. dessen Widerstandsheizelements 6 kann beispielsweise aus einer Klebeverbindung mittels eines elektrisch leitenden Klebstoffes, zum Beispiel mittels eines silberhaltigen Klebers auf Epoxidbasis, bestehen. Die Leiterplatte 4 ragt aus der äußeren Oberfläche des Gehäuses 2 in Form zweier Steckkontakte 8a und 8b heraus. Die beiden Steckkontakte 8a und 8b dienen zur Einleitung der elektrischen Energie in die Inhalatorkomponente. Die elektrische Energie wird dem elektrischen Widerstandsheizelement 6 über Leiterbahnen der Leiterplatte 4 zugeführt. Die Leiterplatte 4 ist vorzugsweise als mehrlagige sogenannte Multilayer-Leiterplatte ausgeführt. Die Leiterbahnen liegen also in mehreren Lagen vor. Die Vorteile dieses speziellen Leiterplatten-Typs wurden bereits früher dargestellt. Die elektrische Energie wird vorzugsweise vom wiederverwendbaren Inhalatorteil bezogen. Das wiederverwendbare Inhalatorteil beinhaltet zu diesem Zweck eine Batterie und einen elektrischen Steuerkreis zur Steuerung der Energiezufuhr.

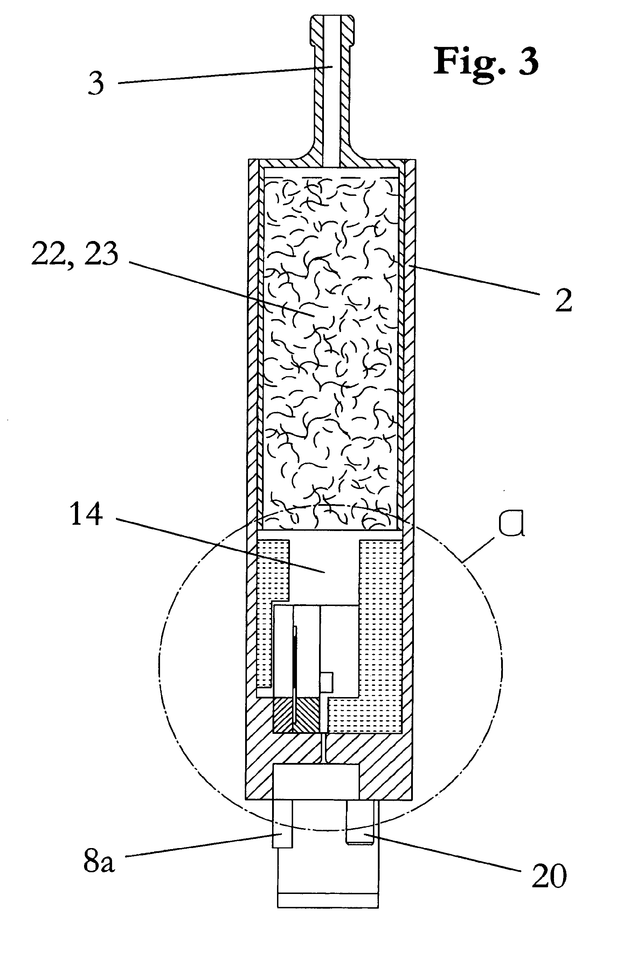

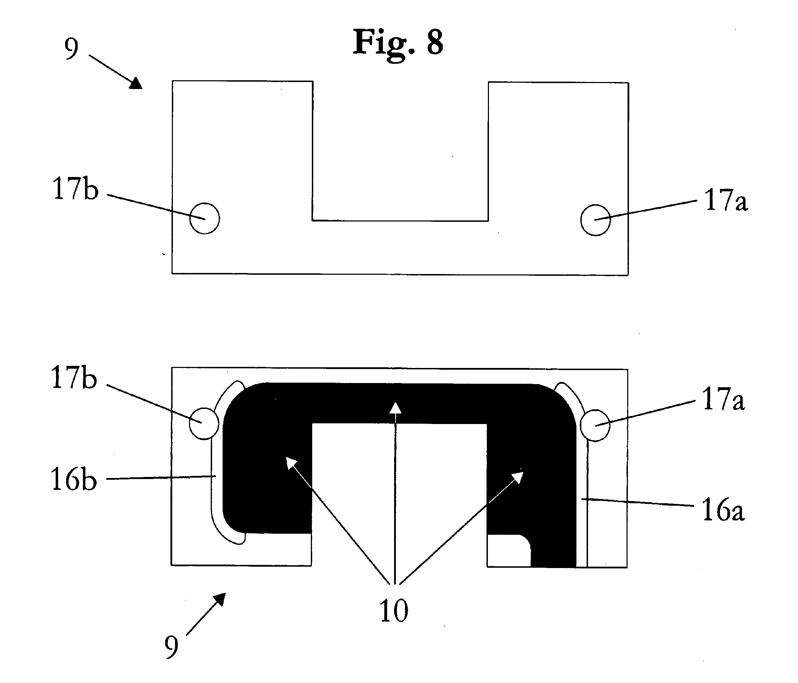

Auf die Leiterplatte 4 ist flächig ein eine Ausnehmung oder Vertiefung 10 aufweisendes Oberteil 9 aufgesetzt - siehe Fig. 3-8. Die Ausnehmung 10 ist in Fig. 8 als schwarze Fläche dargestellt und hat eine Tiefe von typischerweise 0,2mm. Die Ausnehmung 10 ist der Leiterplatte 4 zugewandt und bildet im Zusammenwirken mit deren Oberfläche einen Kapillarspalt aus. Der Kapillarspalt ist in Fig. 2 schematisch als schwarze Fläche dargestellt und setzt sich aus drei Teilabschnitten zusammen: einem ersten Kapillarspalt 11a, in welchen der flächige Verbund 5 bzw. Docht 7 mit seinem Endabschnitt 7a hineinragt; einem zweiten Kapillarspalt 11b, in welchen der flächige Verbund 5 bzw. Docht 7 mit seinem Endabschnitt 7b hineinragt; und einen dritten Kapillarspalt 11c, welcher den ersten Kapillarspalt 11a mit dem zweiten Kapillarspalt 11b verbindet. Der erste Kapillarspalt 11a steht mit einem durch das Gehäuse 2 gebildeten oder in diesem angeordneten Flüssigkeitsbehälter 12 in Verbindung. Der Flüssigkeitsbehälter 12 speichert ein flüssiges Material 13. Die Kapillarkräfte im Kapillarspalt 11a ziehen das flüssige Material 13 aus dem Flüssigkeitsbehälter 12 in den Kapillarspalt 11a hinein. Das flüssige Material 13 erreicht zuerst den Endabschnitt 7a des flächigen Verbundes 5. Dort benetzt das flüssige Material 13 die Kapillarstruktur des Dochts 7, wonach der Docht 7 von dieser Seite her weiter mit flüssigem Material 13 infiltriert werden kann. Parallel fließt das flüssige Material 13 in den Kapillarspalt 11c und gelangt schließlich über diesen bis zum Kapillarspalt 11b, wo es im Endabschnitt 7b die Kapillarstruktur des flächigen Verbundes 5 bzw. des Dochts 7 nochmals benetzt. Der Docht 7 wird also von zwei Seiten her mit dem flüssigem Material 13 infiltriert. Da der Strömungswiderstand der Kapillarspalten wesentlich kleiner ist als der Strömungswiderstand des Dochts 7 verläuft die Infiltration des Dochts 7 von beiden Seiten her fast zeitgleich bzw. symmetrisch. Die Infiltrationsdauer kann im Vergleich zu Anordnungen mit nur einseitiger Versorgung des Dochts 7 (vgl. WO 2010/045671) massiv verkürzt werden.

Nachdem der Docht 7 bzw. flächige Verbund 5 vollständig mit dem flüssigen Material 13 infiltriert ist, kann die elektrische Energie dem elektrischen Widerstandsheizelement 6 über die Leiterbahnen der Leiterplatte 4 zugeführt werden, und das flüssige Material 13 verdampft werden. Damit die Leiterbahnen die Kapillarspalten möglichst nicht tangieren, ist es vorteilhaft, die Leiterbahnen vornehmlich auf der Rückseite der Leiterplatte 4 und gegebenenfalls in Zwischenlayern anzuordnen (Multilayer-Leiterplatte), und die einzelnen Leiterbahnen nach dem Stand der Technik mittels sogenannter Durchkontaktierungen zweckmäßig miteinander zu verbinden. Der freigesetzte Dampf mischt sich in einer im Gehäuse 2 angeordneten Kammer 14 mit der durch eine Lufteinlaßöffnung 15 aus der Umgebung zugeführten Luft (siehe Fig. 3-5) und bildet das Dampf-Luft-Gemisch oder/und Kondensationsaerosol aus, welches hiernach über das Mundstück 3 an einen Benutzer abgegeben werden kann.

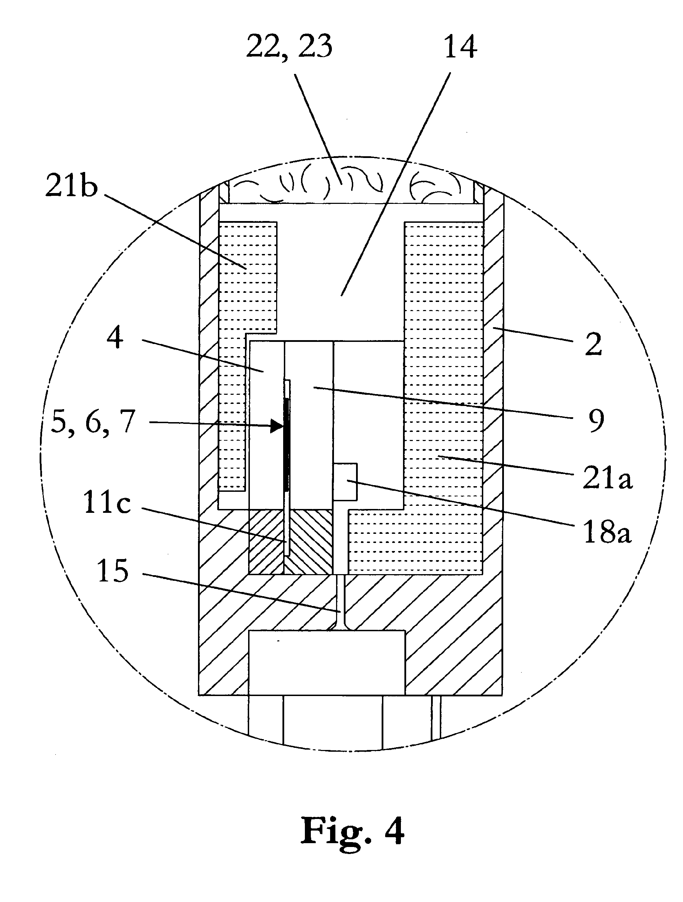

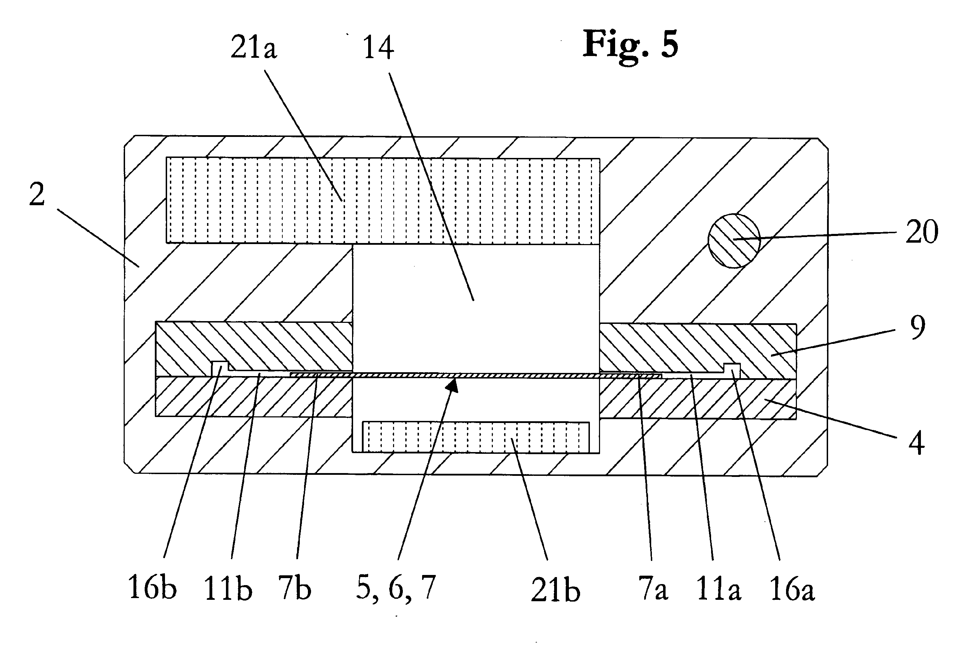

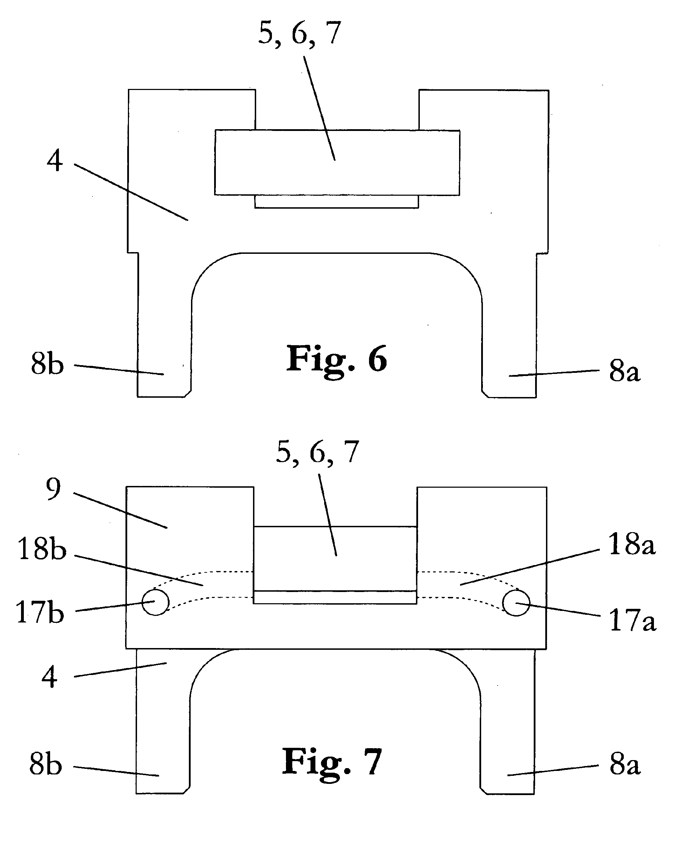

Nach Fig. 8 wird die Ausnehmung 10 im Oberteil 9 im Bereich des ersten Kapillarspalts 11a von einer ersten Belüftungsnut 16a und im Bereich des zweiten Kapillarspalts 11b von einer zweiten Belüftungsnut 16b berandet. In Fig. 2 sind die Belüftungsnuten 16a und 16b schematisch strichliert und in Fig. 5 im Querschnitt dargestellt. Die Belüftungsnut 16a erstreckt sich bis zum Flüssigkeitsbehälter 12 und sorgt dafür, daß jedes aus dem Flüssigkeitsbehälter 12 entnommene Volumen flüssigen Materials 13 durch ein gleich großes Luftvolumen ersetzt wird. Die Belüftungsnuten 16a und 16b beziehen die Luft über durch das Oberteil 9 gebildete Belüftungsbohrungen 17a und 17b, welche ihrerseits über durch das Gehäuse 2 gebildete Verbindungskanäle 18a und 18b mit der Kammer 14 in Verbindung stehen. Die Verbindungskanäle 18a und 18b sind in Fig. 7 schematisch strichliert dargestellt. Die Mündung des Verbindungskanals 18a in die Kammer 14 ist in Fig. 4 dargestellt.

Als Material für die Leiterplatte 4 eignen sich grundsätzlich alle bekannten Leiterplattenwerkstoffe, insbesondere die Werkstofftypen FR1 bis FR5. Das Oberteil 9 ist mit der Leiterplatte 4 durch eine Klebeverbindung gefügt und besteht vorzugsweise ebenfalls aus einem Kunststoff. Wesentlich ist, daß die Oberflächen der Leiterplatte 4 sowie des Oberteils 9 vom flüssigen Material 13 gut benetzt werden. Als flüssiges Material 13 finden bevorzugt hochverdünnte ethanolische oder/und wässrige Lösungen Verwendung, in welchen die eigentlichen Wirkstoffe, aerosolbildenden Stoffe, Aromastoffe, sowie gegebenenfalls weitere Hilfsstoffe gelöst oder/und emulgiert sind. Die Benetzbarkeit und im Übrigen auch die Verklebbarkeit der Kunststoffe kann durch eine Oberflächenaktivierung, beispielsweise duch Hydrophilieren mittels Plasmapolymerisation (Firma Diener electronic GmbH + Co. KG, www.plasma.de) erheblich verbessert werden.

Fig. 9 zeigt eine alternative erfindungsgemäße Ausführungsform der Inhalatorkomponente. Diese Ausführungsform unterscheidet sich von der Anordung gemäß Fig. 2 im Wesentlichen dadurch, daß ein zweiter das flüssige Material 13 enthaltender Flüssigkeitsbehälter 12b vorgesehen ist, welcher mit dem zweiten Kapillarspalt 11b gekoppelt bzw. koppelbar ist. Tritt im ersten Versorgungspfad (Flüssigkeitsbehälter 12a und Kapillarspalt 11a) eine Störung der Flüssigkeitsversorgung auf, so kann der flächige Verbund 5 bzw. dessen Docht 7 immer noch über den zweiten Versorgungspfad (Flüssigkeitsbehälter 12b, Kapillarspalt 11b und ggf. Kapillarspalt 11c) ausreichend mit flüssigem Material 13 versorgt werden.

Im Folgenden sollen noch weitere Bestandteile der Inhalatorkomponente kurz beschrieben werden. Auch wenn diese Bestandteile nicht unmittelbar erfindungsrelevant sein mögen, so trägt die Beschreibung derselben doch dazu bei, die Funktion der erfindungsgemäßen Inhalatorkomponente im Ganzen noch besser zu verstehen, und die Ausführbarkeit der Erfindung noch sicherer zu gewährleisten: wie Fig. 2 exemplarisch zeigt, weist der Flüssigkeitsbehälter 12 an einer Stirnseite einen klappenartigen, öffenbaren Verschluß 19 auf. Der öffenbare Verschluß 19 schließt in seiner Schließstellung den Flüssigkeitsbehälter 12 nach außen hin hermetisch ab. Erst nach Öffnen des öffenbaren Verschlusses 19 kann das flüssige Material 13 den Kapillarspalt 11a benetzen und durch die im Kapillarspalt wirkenden Kapillarkräfte sodann weiter bis zum flächigen Verbund 5 vordringen und schließlich den Docht 7 des flächigen Verbundes 5 infiltrieren. Der öffenbare Verschluß 19 wird mit Hilfe eines im Gehäuse 2 axial verschiebbar gelagerten Stiftes 20 geöffnet (Fig. 3 und Fig. 5). Ein erstes Ende des Stiftes 20 ist gegen den öffenbaren Verschluß 19 gerichtet. Ein zweites Ende ragt bei noch geschlossenem Verschluß 19 aus der äußeren Oberfläche des Gehäuses 2 fortsatzartig heraus. Das zweite Ende des Stiftes 20 steht mit dem wiederverwendbaren Inhalatorteil in einer stößelartigen Wirkverbindung. Im Zuge der Kopplung der Inhalatorkomponente mit dem wiederverwendbaren Inhalatorteil wird der Stift 20 in das Gehäuse 2 verschoben, wodurch das erste Ende des Stiftes 20 gegen den öffenbaren Verschluß 19 drückt. Der öffenbare Verschluß 19 weist auf seinem Umfang eine Materialschwächung auf, welche so dimensioniert ist, daß sie bei Druckbeaufschlagung durch den Stift 20 gleich einer Sollbruchstelle über einen weiten Umfangsbereich aufreißt, jedoch auf einer Seite ein Scharnier ausbildet. Auf diese Weise wird bewirkt, daß sich der öffenbare Verschluß 19 wie eine Klappe öffnet.

Fig. 2-5 zeigen ferner eine in der Kammer 14 angeordnete Kondensatbindeeinrichtung bestehend aus zwei offenporigen, saugfähigen Schwämmen 21a und 21b. Die Schwämme 21a und 21b nehmen in ihren Poren aus der Dampfphase gebildete Kondensatablagerungen auf und verhindern, daß sich in der Inhalatorkomponente insbesondere in der Kammer 14 frei bewegliche Kondensatansammlungen bilden, welche die Funktion der Inhalatorkomponente beeinträchtigen könnten, aber auch ein Risiko für den Benutzer und die Umwelt darstellen, sofern diese Ansammlungen Arzneimittelreste oder Gifte wie Nikotin enthalten. Die beiden Schwämme 21a und 21b kleiden die Innenwände der Kammer 14 weitgehend aus, wobei sich der Schwamm 21a bis zur Mündung der Lufteinlaßöffnung 15 erstreckt. Hierdurch soll verhindert werden, daß Kondensatablagerungen in die relativ enge schlitzförmige Lufteinlaßöffnung 15 gelangen, wodurch die Luftströmung behindert werden könnte. In einer alternativen Anordnung könnte die Lufteinlaßöffnung 15 auch unmittelbar durch die Schwämme 21a und 21b gebildet werden. Die Schwämme 21a und 21b bestehen vorzugsweise aus feinporigen, hochporösen Faserverbunden. Die Firma Filtrona Richmond Inc., www.filtronaporoustechnologies.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet werden.

Wie Fig. 2-3 zeigen, ist den Schwämmen 21a und 21b stromabwärts ein Kühler 22 nachgeordnet, welcher im konkreten Ausführungsbeispiel in das vorzugsweise auswechselbare Mundstück 3 integriert ist und aus einem porösen Füllmaterial 23 besteht, dessen Poren vom gebildeten Dampf-Luft-Gemisch oder/und Kondensationsaerosol durchströmt werden. Der Kühler 22 kühlt das durchströmende Dampf-Luft-Gemisch oder/und Kondensationsaerosol und entzieht ihm hierbei noch weiteres Kondensat. Hierdurch können die organoleptischen Eigenschaften des vom Benutzer aufgenommenen Dampf-Luft-Gemisches oder/und Kondensationsaerosols deutlich verbessert werden. Das Füllmaterial 23 kann beispielsweise aus einer Tabakfüllung bestehen. Eine solche Tabakfüllung bewirkt zusätzlich eine Aromatisierung des durch sie hindurchströmenden Dampf-Luft-Gemisches bzw. Kondensationsaerosols und bietet sich vor allem dann an, wenn das flüssige Material 13 Nikotin enthält.

Abschließend sei darauf hingewiesen, daß die Erfindung selbstverständlich nicht auf einen flächigen Verbund 5 gemäß den soeben beschriebenen Ausführungsbeispielen beschränkt ist. Der Verbund könnte ebenso linienförmig ausgebildet sein. Ferner könnte sich der Verbund auch aus mehreren nebeneinander angeordneten Verbunden oder Verbundabschnitten zusammensetzen, wobei es unerheblich ist, wie die einzelnen Verbunde bzw. Verbundabschnitte miteinander elektrisch verschaltet sind. In diesem Zusammenhang sei angemerkt, daß sich mittels der erfindungsgemäßen Multilayer-Leiterplatte 4 Reihenschaltungen, Parallelschaltungen sowie noch komplexere Verschaltungen und Ansteuerungen realisieren lassen. Schließlich umfaßt die Erfindung auch Vorrichtungen, in welchen das Heizelement vom Docht getrennt angeordnet ist. Beispielsweise könnte der Docht flächig ausgebildet sein, und die Heizenergie durch elektromagnetische Wellen, insbesondere Wärmestrahlung oder Mikrowellen auf den Docht übertragen werden.

### Bezugszeichenliste

- 1: Schnappverbindung
- 2: Gehäuse
- 3: Mundstück
- 4: Trägerplatte, Leiterplatte
- 5: flächiger Verbund
- 6: Heizelement, Widerstandsheizelement
- 7: Docht
- 7a, 7b: Endabschnitte des Dochts bzw. Verbundes
- 8a, 8b: Steckkontakte
- 9: Oberteil
- 10: Ausnehmung
- 11a: erster Kapillarspalt
- 11b: zweiter Kapillarspalt
- 11c: dritter Kapillarspalt
- 12: Flüssigkeitsbehälter
- 12a: erster Flüssigkeitsbehälter
- 12b: zweiter Flüssigkeitsbehälter
- 13: flüssiges Material
- 14: Kammer
- 15: Lufteinlaßöffnung
- 16a, 16b: Belüftungsnuten
- 17a, 17b: Belüftungsbohrungen
- 18a, 18b: Verbindungskanäle
- 19: öffenbarer Verschluß
- 20: Stift
- 21a, 21b: Schwämme
- 22: Kühler
- 23: Füllmaterial

## Patentansprüche

1. Inhalatorkomponente für die Bildung eines Dampf-Luft-Gemisches oder/und Kondensationsaerosols durch Verdampfung eines flüssigen Materials (13) und gegebenenfalls Kondensation des gebildeten Dampfes, umfassend:
ein Heizelement (6) zur Verdampfung einer Portion des flüssigen Materials (13);
einen Docht (7) zur selbsttätigen Versorgung des Heizelements (6) mit dem flüssigen Material (13), welcher Docht (7) zumindest zwei voneinander entfernt angeordnete Endabschnitte (7a, 7b) aufweist;
einen ersten Kapillarspalt (11a) zur selbsttätigen Versorgung des Dochts (7) mit dem flüssigen Material (13), wobei ein erster Endabschnitt (7a) des Dochts (7) in den ersten Kapillarspalt (11a) ragt,
**gekennzeichnet durch** einen zweiten Kapillarspalt (11b), welcher den zweiten Endabschnitt (7b) des Dochts (7) in sich aufnimmt.

2. Inhalatorkomponente nach Anspruch 1, **dadurch gekennzeichnet, daß** der erste und zweite Kapillarspalt (11a, 11b) über einen dritten Kapillarspalt (11c) miteinander verbunden sind.

3. Inhalatorkomponente nach Anspruch 2, **dadurch gekennzeichnet, daß** einer der Kapillarspalten (11a, 11b, 11c) mit einem das flüssige Material (13) enthaltenden Flüssigkeitsbehälter (12) kapillar gekoppelt bzw. koppelbar ist.

4. Inhalatorkomponente nach Anspruch 1 oder 2, **dadurch gekennzeichnet, daß** sämtliche Kapillarspalten (11a, 11b, 11c) in einer gemeinsamen Ebene angeordnet sind.

5. Inhalatorkomponente nach Anspruch 4, **dadurch gekennzeichnet, daß** sämtliche Kapillarspalten (11a, 11b, 11c) durch eine Platte, vorzugsweise Leiterplatte (4), und ein auf die Platte aufgesetztes Oberteil (9) gebildet werden.

6. Inhalatorkomponente nach Anspruch 5, **dadurch gekennzeichnet, daß** das Oberteil (9) eine der Platte zugewandte Ausnehmung (10) aufweist.

7. Inhalatorkomponente nach Anspruch 6, **dadurch gekennzeichnet, daß** die Ausnehmung (10) zumindest abschnittsweise von einer oder mehreren Belüftungsnuten (16a, 16b) berandet wird.

8. Inhalatorkomponente nach Anspruch 5, **dadurch gekennzeichnet, daß** das Oberteil (9) auf den Endabschnitten (7a, 7b) des Dochts (7) lagert.

9. Inhalatorkomponente nach Anspruch 5, **dadurch gekennzeichnet, daß** die Leiterplatte (4) als mehrlagige sogenannte Multilayer-Leiterplatte ausgeführt ist.

10. Inhalatorkomponente nach Anspruch 1 oder 2, **dadurch gekennzeichnet, daß** der erste Kapillarspalt (11a) mit einem das flüssige Material (13) enthaltenden ersten Flüssigkeitsbehälter (12a) gekoppelt bzw. koppelbar ist, und der zweite Kapillarspalt (11b) mit einem das flüssige Material (13) enthaltenden zweiten Flüssigkeitsbehälter (12b) gekoppelt bzw. koppelbar ist.

11. Inhalator, umfassend eine Inhalatorkomponente nach einem der Ansprüche 1-10.

## Claims

1. Inhaler component for forming a vapour-air mixture and/or condensation aerosol by vaporizing a liquid material (13) and optionally condensing the vapour formed, comprising:
a heating element (6) for vaporizing a portion of the liquid material (13);
a wick (7) for automatically supplying the liquid material (13) to the heating element (6), said wick (7) having at least two end portions (7a, 7b) arranged apart from each other;
a first capillary gap (11a) for automatically supplying the liquid material (13) to the wick (7), a first end portion (7a) of the wick (7) protruding into the first capillary gap (11a),
**characterized by** a second capillary gap (11b), which receives within it the second end portion (7b) of the wick (7).

2. Inhaler component according to Claim 1, **characterized in that** the first and second capillary gaps (11a, 11b) are connected to each other by a third capillary gap (11c).

3. Inhaler component according to Claim 2, **characterized in that** one of the capillary gaps (11a, 11b, 11c) is coupled or can be coupled in a capillary manner to a liquid container (12) containing the liquid material (13).

4. Inhaler component according to Claim 1 or 2, **characterized in that** all the capillary gaps (11a, 11b, 11c) are arranged in a common plane.

5. Inhaler component according to Claim 4, **characterized in that** all the capillary gaps (11a, 11b, 11c) are formed by a board, preferably a printed circuit board (4), and an upper part (9) mounted on the board.

6. Inhaler component according to Claim 5, **characterized in that** the upper part (9) has a recess (10) facing towards the board.

7. Inhaler component according to Claim 6, **characterized in that** the recess (10) is at least partially edged by one or more ventilation grooves (16a, 16b).

8. Inhaler component according to Claim 5, **characterized in that** the upper part (9) bears on the end portions (7a, 7b) of the wick (7).

9. Inhaler component according to Claim 5, **characterized in that** the printed circuit board (4) is designed as a multi-layer printed circuit board.

10. Inhaler component according to Claim 1 or 2, **characterized in that** the first capillary gap (11a) is coupled or can be coupled to a first liquid container (12a) containing the liquid material (13), and the second capillary gap (11b) is coupled or can be coupled to a second liquid container (12b) containing the liquid material (13).

11. Inhaler, comprising an inhaler component according to one of Claims 1-10.

## Revendications

1. Elément inhalateur pour la formation d'un mélange de vapeur et d'air et/ou d'un aérosol de condensation par évaporation d'un matériau fluide (13) et éventuellement condensation de la vapeur formée, comprenant :
un élément chauffant (6) pour l'évaporation d'une portion du matériau fluide (13) ;
une mèche (7) pour l'alimentation automatique de l'élément chauffant (6) en le matériau fluide (13), laquelle mèche (7) présente au moins deux portions d'extrémité (7a, 7b) disposées à distance l'une de l'autre ;
une première fente capillaire (11a) pour l'alimentation automatique de la mèche (7) en le matériau fluide (13), une première portion d'extrémité (7a) de la mèche (7) pénétrant dans la première fente capillaire (11a),
**caractérisé par** une deuxième fente capillaire (11b) qui reçoit en elle la deuxième portion d'extrémité (7b) de la mèche (7).

2. Elément inhalateur selon la revendication 1, **caractérisé en ce que** la première et la deuxième fente capillaire (11a, 11b) sont connectées l'une à l'autre par le biais d'une troisième fente capillaire (11c).

3. Elément inhalateur selon la revendication 2, **caractérisé en ce que** l'une des fentes capillaires (11a, 11b, 11c) est accouplée ou peut être accouplée par voie capillaire à un récipient de liquide (12) contenant le matériau fluide (13).

4. Elément inhalateur selon la revendication 1 ou 2, **caractérisé en ce que** toutes les fentes capillaires (11a, 11b, 11c) sont disposées dans un plan commun.

5. Elément inhalateur selon la revendication 4, **caractérisé en ce que** toutes les fentes capillaires (11a, 11b, 11c) sont formées par une plaque, de préférence une carte à circuits imprimés (4), et par une partie supérieure (9) placée sur la plaque.

6. Elément inhalateur selon la revendication 5, **caractérisé en ce que** la partie supérieure (9) présente un évidement (10) tourné vers la plaque.

7. Elément inhalateur selon la revendication 6, **caractérisé en ce que** l'évidement (10) est bordé au moins en partie par une ou plusieurs rainures de ventilation (16a, 16b).

8. Elément inhalateur selon la revendication 5, **caractérisé en ce que** la partie supérieure (9) s'appuie sur les portions d'extrémité (7a, 7b) de la mèche (7).

9. Elément inhalateur selon la revendication 5, **caractérisé en ce que** la carte à circuits imprimés (4) est réalisée sous forme de carte à circuits imprimés multicouche dite Multilayer.

10. Elément inhalateur selon la revendication 1 ou 2, **caractérisé en ce que** la première fente capillaire (11a) est accouplée ou peut être accouplée à un premier récipient de liquide (12a) contenant le matériau fluide (13), et la deuxième fente capillaire (11b) est accouplée ou peut être accouplée à un deuxième récipient de liquide (12b) contenant le matériau fluide (13).

11. Inhalateur, comprenant un élément inhalateur selon l'une quelconque des revendications 1 à 10.
